(19) Europäisches Patentamt
European Patent Office
Office européen des brevets

(11) Publication number: 0 319 111 A2

# (12) EUROPEAN PATENT APPLICATION

(21) Application number: 88202745.1

(51) Int. Cl.[4]: C07C 127/19

(22) Date of filing: 29.11.88

(30) Priority: 04.12.87 GB 8728397

(43) Date of publication of application: 07.06.89 Bulletin 89/23

(84) Designated Contracting States: AT BE CH DE ES FR GB GR IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Goodall, Brian Leslie
Badhuisweg 3
NL-1031 CM Amsterdam(NL)
Inventor: Terlouw, Willem
Badhuisweg 3
NL-1031 CM Amsterdam(NL)

(74) Representative: Hunter, Keith Roger Ian et al
4 York Road
London SE1 7NA(GB)

## (54) Process for the preparation of phenyl ureas.

(57) A process for the preparation of phenyl ureas, which comprises reacting a nitrobenzene with an amine and carbon monoxide at an elevated temperature in the presence of a palladium catalyst, the amine being supplied continuously or stepwise during the period of the reaction.

EP 0 319 111 A2

# PROCESS FOR THE PREPARATION OF PHENYL UREAS

This invention relates to a process for the preparation of phenyl ureas and is particularly, although not exclusively, concerned with the preparation of phenyl ureas suitable for use as herbicides.

The phenyl ureas are a known class of commercially available herbicides. There is continuing interest in new methods of preparing such ureas and, especially, in processes which can be operated under comparatively mild conditions without using environmentally unacceptable reagents such as phosgene.

Dieck et al, J. Org. Chem, 40 (19), 1975, p 2819-2822 describe a process for the preparation of phenyl ureas, which comprises reacting an aromatic nitro compound with an aromatic primary amine and carbon monoxide at an elevated temperature in the presence of a palladium catalyst. In the process, all of the aromatic nitro compound and the aromatic primary amine was present at the start of the reaction.

Surprisingly, it has now been found that the yield of phenyl ureas can be increased by supplying amine continuously or stepwise during the period of the reaction.

Accordingly, the present invention provides a process for the preparation of phenyl ureas, which comprises reacting a nitrobenzene with an amine and carbon monoxide at an elevated temperature in the presence of a palladium catalyst, the amine being supplied continuously or stepwise during the period of the reaction.

The amine is preferably supplied continuously during the period of the reaction.

The preferred rate of supply of amine will depend upon the particular reactants and reaction conditions selected, and may readily be determined by routine experimentation.

Preferably the amine will be supplied over at least 40%, more preferably at least 50% of the period of the reaction. In certain cases it may be advantageous to supply the amine over at least 80%, or even up to 90% of the period of the reaction.

The amine may be supplied in an amount less than the molar amount of nitrobenzene. However, the amine is preferably supplied in an amount at least equal to the molar amount of nitrobenzene.

The carbon monoxide is preferably supplied by flushing the gas through the reaction mixture during the period of the reaction. When carbon monoxide is supplied by flushing the gas through the reaction mixture during the period of the reaction, and the amine is volatile, it is advantageous to supply the amine in an amount in excess of the molar amount of nitrobenzene.

The process is preferably effected at a temperature in the range of from 70°C to 160°C, more preferably 100° to 140°C dependent on the reaction solvent and stability of the catalyst used. The process may be effect at atmospheric pressure. However, it is preferably carried out at elevated pressures, for example at least 5 bar, more preferably from 10 to 150 bar.

If a solvent is employed in the reaction, the solvent may be any unreactive organic solvent such as a halogenated hydrocarbon, for example chloroform, 1,2-dichloroethane, chlorobenzene and the three dichlorobenzenes; a hydrocarbon such as hexane, cyclohexane, octane, benzene, toluene and the three xylenes; an ether such as diethyl ether, tetrahydrofuran, dioxane and anisole; an amide such as N,N-dimethylformamide; and an ester, such as ethyl acetate. A particularly preferred solvent is 1,2-dichlorobenzene.

The palladium catalyst may comprise palladium or a palladium compound. Palladium can be used as metal, deposited on an inert carrier, such as carbon or alumina, or in the form of palladium compounds.

Preferably the palladium catalyst comprises a palladium salt or complex. Examples of palladium salts include palladium chloride, palladium bromide, palladium iodide, palladium carboxylates such as palladium acetate, palladium propionate and palladium isobutyrate. Palladium carboxylates are preferred, particularly palladium acetate. Examples of palladium complexes include palladium acetylacetonate, sodium tetrachloro palladate, potassium tetrachloropalladate, potassium tetraiodopalladate, and complexes containing carbon monoxide such as (tetrabutyl ammonium)$Pd(CO)Cl_3$.

The amount of palladium used in the process of the invention is not critical, but is conveniently between 0.001%w and 10%w, in particular between 0.005%w and 3%w, based on the amount of nitrobenzene present.

When the palladium catalyst comprises a palladium salt or complex, preferably it further comprises a salt of a metal selected from copper, iron, manganese, vanadium, chromium, zinc, tin, uranium and cerium. Examples of salts of these metals include chlorides, bromides and iodides, sulphates, hydrocarbyl sulphonates, fluoroborates and perchlorates. Salts of copper and vanadium are preferred, in particular copper hydrocarbylsulphonates such as copper tosylate and vanadium halides such as vanadium trichloride. The molar ratio of palladium compound to salt of metal is preferably in the range of from 0.01 to 10.

When the palladium catalyst comprises a palladium salt or complex, it preferably also comprises, as promoter, a ligand which is an organo-nitrogen or organo-phosphorus compound having a lone pair of electrons.

Examples of organo-nitrogen compounds which can be used conveniently include mono-, bi- or polycyclic systems containing one or more, especially 1 or 2, nitrogen atoms, for example pyrrole, pyrrolidine, pyridine, piperidine, pyrimidine, 1,10-phenanthroline, pyrazine, benztriazole, morpholine, 2,2′-bipyridyl, 2,2′-biquinoline, bis-pyridylketone, bis-pyridylglyoxal, and their alkyl-substituted derivatives. Analogues containing other substituents, for example sulphonyl, carbonyl, alkoxy and halide moieties, may also be used. Preferred are pyridine and alkyl-substituted pyridines such as the various picolines, e.g. alpha-picoline, 2,2′-bipyridyl and 1,10-phenanthroline. Non-cyclic amines may also be used, for example tetraethylenediamine, 1,2-bis(dimethylamino)-ethane, 1,2-bis(diethylamino)ethane, 1,2-bis(dimethylamino)-propane, 1,2-bis(di-t.-butylamino)ethane, 1,2-bis(diphenylamino)propane and 1,2-bis(diphenylamino)butane.

Examples of organo-phosphorus compounds which can conveniently be used include phosphites and tertiary phosphines of the general formula $P.OR^a.OR^b.OR^c$ or $PR^aR^bR^c$, wherein each of $R^a$, $R^b$ and $R^c$ independently represents an alkyl, cycloalkyl, aryl, alkaryl or aralkyl group having up to 10 carbon atoms, optionally substituted by one or more substituents selected from fluorine and chlorine atoms, alkoxy and aryloxy groups, in particular methoxy and phenoxy groups, cyano groups, and groups of formula $-PR^aR^b$; or $R^a$ and $R^b$ together with the interjacent heteroatom together represent a ring. Preferred monodentate organo-phosphorus compounds include trimethylphosphine, triethylphosphine, tri-n-butylphosphine and triphenylphosphine. Examples of bidentate phosphorus-containing ligands are 2-dimethyl-2-(methyldiphenylphosphino)-1,3-di(diphenyl-phosphino) propane, tetramethyl diphosphinoethane, tetramethyl diphosphinopropane, tetraethyl diphosphinoethane, tetrabutyl diphosphinoethane, dimethyl diethyl diphosphinoethane, tetraphenyl diphosphinoethane, tetraperfluorophenyl diphosphinoethane, tetraphenyl diphosphinopropane, tetraphenyl diphosphinobutane, dimethyl diphenyl diphosphinoethane, diethyl diphenyl diphosphinopropane, tetratolyl diphosphinoethane, ditolyl diphenyl diphosphinoethane, tetratrifluoromethyl diphosphinotetrafluoroethane, tetraphenyl diphosphinoethene and derivatives thereof and 1,2-bis-(diphenylphosphino)benzene and derivatives thereof. Preferred are tetraphenyldiphosphino- butane, tetraphenyl diphosphinoethane and tetraphenyl diphosphinopropane.

Preferred promotors are bidentate ligands containing nitrogen and/or phosphorus.

The ligand, when present, is preferably present in an amount such that the molar ratio of ligand to palladium compound is in the range of from 0.1 to 100.

When the palladium catalyst comprises a palladium salt or complex, it preferably comprises a derivative of an acid having a pKa of less than 2, other than a hydrohalic acid. Preferred examples include hydrocarbylsulphonates (e.g. tosylates), sulphates, fluoroborates and perchlorates. These derivatives may conveniently be present in the form of salts of palladium or of the metals given above. Alternatively, they may be present in the form of the parent acids.

Phenyl ureas which may be prepared by the process according to the invention include compounds of formula

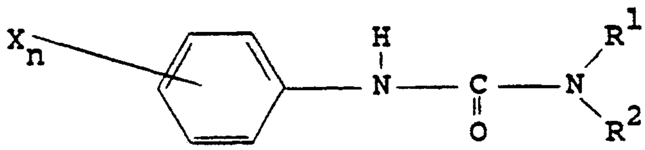

(I)

wherein $R^1$ represents an optionally substituted alkyl, alkenyl, cycloalkyl, aryl or heterocyclyl group, $R^2$ represents a hydrogen atom or an optionally substituted alkyl, alkenyl, cycloalkyl, aryl or heterocyclyl group, the or each X represents a halogen atom, or an optionally substituted alkyl, alkoxy, aryl or aryloxy group and n is O or an integer from 1 to 5. Such compounds are prepared by the process according to the invention when the nitrobenzene is a compound of formula II

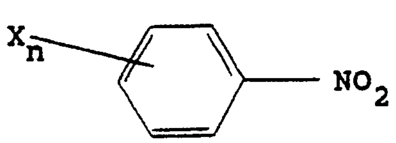

(II)

where X and n are as defined above, and the amine is a compound of formula $R^1R^2NH$ where $R^1$ and $R^2$ are as defined above.

In the process according to the invention, the amine is a primary or secondary amine. It is preferably an alkylamine or a dialkylamine.

When the nitrobenzene or the amine contains an alkyl group, this may be linear or branched and may contain up to 10, preferably up to 6 carbon atoms, for example methyl or ethyl. When the nitrobenzene or the amine contains a haloalkyl group, this usually contains up to 6, preferably up to 4, carbon atoms and the halogen atom is suitably fluorine, bromine or chlorine. Trifluoromethyl is the preferred haloalkyl group. When a substituent is described as being optionally substituted, the substituent groups which are optionally present may be for example any of those customarily employed in the development of pesticidal compounds. Specific examples include halogen, especially chlorine, atoms and nitro, cyano, alkyl and haloalkyl group, especially trifluoromethyl groups.

Preferably, $R^1$ in formula (I) is a methyl group and $R^2$ is hydrogen atom or a methyl group. n is preferably 1 or 2 and preferred substituents X include chlorine, fluorine and bromine atoms, alkyl groups (eg. isopropyl), trifluoromethyl and alkoxy (eg. methoxy) or aryloxy, groups.

Starting nitrobenzenes and amines are commercially available compounds or can be readily obtained by standard preparative methods.

The following Examples illustrate the invention. Products were identified by high performance liquid chromatography (HPLC) via comparison with authentic reference samples.

## Preparation of Catalyst

Catalyst A - $Pd(OCOCH_3)_2.Cu(tosyl)_2$.bipyridyl Copper (II) tosylate was prepared as follows:

To a stirred solution of p-toluenesulphonic acid (20g) in water (500 ml) at 50 °C was slowly added powdered copper (II) hydroxide, whilst monitoring the pH of the solution. Addition was stopped when the pH reached 7 (after addition of about 10 g of hydroxide). After cooling to ambient temperature the solution was filtered and the filtrate evaporated to dryness. The solid residue was vacuum oven dried at 40 °C to give green-blue crystals of copper (II) tosylate (21 g) containing approximately 10% water.

Copper (II) tosylate, and commercially available palladium acetate and bipyridyl were combined in the molar ratio 1: 0.5:10 to give catalyst A.

Catalyst B - $(Bu_4N)Pd(CO)Cl_3 + VCl_3.3THF$

The palladium complex was prepared as described by Browning et al, J. Chem. Soc. Dalton Trans., (1977), 2061. $VCl_3$.3THF was prepared by dissolving $VCl_3$ in boiling tetrahydrofuran followed by evaporation to dryness.

The palladium complex and vanadium complex were combined in the molar ratio 1:5 to give Catalyst B.

## Example 1

### Preparation of 1,1-dimethyl-3(4-chlorobenzene)-urea

A) Comparative Example

A solution of dimethylamine (100 mmoles: 4.5 g) in 1,2-dichlorobenzene (15 ml) was added to a mixture of 1,2-dichlorobenzene (85 ml) and 4-chloronitrobenzene (100 mmoles; 15.85 g), containing as catalyst Pd $(OCOCH_3)_2$ (0.5 mmol; 0.112 g), copper (II) tosylate (1 mmol; 0.405 g) and bipyridyl (10 mmol; 1.68 g). Carbon monoxide was flushed through the reaction mixture at a flow rate of 3 Nl/h under the conditions of pressure, temperature and time given in Table 1 below.

B) Example illustrating the process according to the invention.

Run A above was repeated with differing conditions of pressure, temperature and time and with the dimethylamine addition staged over 15 hours.

C) Example illustrating the process according to the invention.

Run A) above was repeated using dimethylamine (130 mmoles; 5.85 g) and the conditions of pressure, temperature and time given in Table 1.

The products of Runs A) to C), ie (1,1-dimethyl-3(4-chlorobenzene)-urea and p-chloroaniline were separated by filtering the precipitated urea and identified by HPLC and/or GLC. The yields are given in Table 1 below.

Table 1

| Run | Pressure (bar) | Time (hours) | Temp (°C) | Conversion molar (%) | Urea molar (%) | Aniline molar (%) |
|---|---|---|---|---|---|---|
| A* | 40 | 3 | 120 | 99 | 22 | 41 |
| B | 10 | 18 | 100 | >99 | 41 | 31 |
| C | 50 | 20 | 140 | >99 | 73 | 17 |

* Comparative Example.

Example 2

Preparation of 1,1-dimethyl-3(4-chlorobenzene)-urea

Tetrabutylammonium chloride (0.39mmol, 0.107 g), $(Bu_4N)Pd(CO)Cl_3$ (0.32 mmol; 0.156 g) and $VCl_3.3THF$ (1.5 m mole; 0.56 g) were dissolved in 1,2-dichlorobenzene (70 ml). 4-Chloronitrobenzene (100 mmol; 15.85 g) was added and the mixture heated to 135°C. Carbon monoxide was flushed through the reaction mixture at 50 bar pressure at a flow rate of 3 Nl/hour. A solution of dimethylamine (120 m.mol; 5.4g) in 1,2-dichlorobenzene (20 ml) was added slowly over 17 hours. After 18 hours, the conversion of starting material was 70%. The desired product (1,1-dimethyl-3(4-chlorobenzene)urea was isolated by filtration as a 99% pure solid.

Example 3

Preparation of 1,1-dimethyl-3(4-isopropylbenzene)-urea

To a solution of 4-isopropyl-nitrobenzene (100 mmole; 16.5 g) in 1,2-dichlorobenzene (80 ml) containing as catalyst Pd $(OCOCH_3)_2$ (0.5 mmol; 0.112 g), copper (II) tosylate (1 m mol; 0.405g) and bipyridyl (10 mmol; 1.68 g) were added gradually a solution of dimethylamine (115 mmol; 5.18 g) in 1,2-dichlorobenzene (20ml). The addition was carried out over a period of 20 hours at 130°C and with carbon monoxide flow through the reactor of 2Nl/hour at a pressure of 50 bar, stirring was then continued for a further 4 hours. Analysis of the reaction mixture showed a 75% conversion of the starting material of which 53% was the desired product 1,1-dimethyl-3(4-isopropyl- benzene)-urea.

Example 4

1-n-butyl,3-(4-chlorobenzene)-urea

To 4-chloronitrobenzene (100 mmol; 15.85 g) in 1,2-dichlorobenzene (100ml) containing a catalyst of $Pd(OCOCH_3)_2$(0.5 mmol; 0.112 g), copper (II) tosylate (1 mmol; 4.405 g) and bipyridyl (10 mmol; 1.68 g) was added over a period of 12 hours n-butylamine (80 mmol; 5.84 g). The reaction was stirred and maintained at 130 °C with carbon monoxide passage of 3 Nl/hour at a pressure of 50 bar. After amine addition the reaction was allowed to proceed for a further 10 hours. Conversion of the starting material was complete and the yield of the desired product 1-n-butyl,3-(4-chlorobenzene) urea was found to be 61%.

In a comparative experiment in which the n-butylamine was added in one dose at the beginning of a 4-hour reaction period, analysis revealed that although there was 90% conversion of starting material, the major product was 4-chloroaniline with only a minor amount (18%) of 1-n-butyl,3-(4-chlorobenzene)-urea.

## Claims

1. A process for the preparation of phenyl ureas, which comprises reacting a nitrobenzene with an amine and carbon monoxide at an elevated temperature in the presence of a palladium catalyst, characterised in that the amine is supplied continuously or stepwise during the period of the reaction.

2. A process as claimed in claim 1, characterised in that the amine is supplied continuously.

3. A process as claimed in claim 1 or claim 2, characterised in that the amine is supplied over at least 50% of the period of the reaction.

4. A process as claimed in any one of claims 1 to 3, characterised in that the palladium catalyst comprises a salt or complex of palladium.

5. A process as claimed in claim 4, characterised in that the palladium catalyst comprises a salt of a metal selected from copper, iron, manganese, vanadium, chromium, zinc, tin, uranium and cerium.

6. A process as claimed in claim 4 or claim 5, characterised in that the palladium catalyst comprises a ligand which is an organo-nitrogen or organo-phosphorus compound having a lone pair of electrons.

7. A process as claimed in any one of claims 4 to 6, characterised in that the palladium catalyst comprises a hydrocarbylsulphonate, a sulphate, a fluoroborate or a perchlorate.

8. A process as claimed in any one of claims 1 to 7, characterised in that the temperature is in the range of from 70 to 160 °C.

9. A process as claimed in any one of claims 1 to 8, characterised in that the pressure is in the range of from 10 to 150 bar.

10. A process as claimed in any one of claims 1 to 9, characterised in that the carbon monoxide is flushed through the reaction mixture continuously during the reaction period.

11. A process as claimed in any one of claims 1 to 10, characterised in that the reaction is effected in the presence of a chlorobenzene as solvent.

12. A process as claimed in any one of claims 1 to 11, characterised in that the amine is an alkylamine or a dialkylamine.